# EUROPEAN PATENT SPECIFICATION

(11) **EP 2 381 886 B1**
(45) Date of publication and mention of the grant of the patent: **28.08.2019**
(21) Application number: 09836679.2
(22) Date of filing: 07.12.2009
(51) Int. Cl.: A61B 17/00, A61B 17/12

(54) **BRAIDED OCCLUSION DEVICE HAVING REPEATING EXPANDED VOLUME SEGMENTS SEPARATED BY ARTICULATION SEGMENTS**
GEFLOCHTENE OKKLUSIONSVORRICHTUNG MIT MEHREREN DURCH GELENKSEGMENTE GETRENNTE EXPANDIERTEN VOLUMENSEGMENTEN
DISPOSITIF D'OCCLUSION TRESSÉ DOTÉ DE SEGMENTS RÉPÉTÉS À VOLUME EXPANSÉ SÉPARÉS PAR DES SEGMENTS D'ARTICULATION

(30) Priority: 30.12.2008 US 346073
(43) Date of publication of application: 02.11.2011
(73) Proprietor: St. Jude Medical, Cardiology Division, Inc., St. Paul, MN 55117 (US)
(72) Inventor: QIAN, Zhong, Maple Grove, MN 55311 (US); ADAMS, Daniel, O., Long Lake, MN 55356 (US)
(74) Representative: Potter Clarkson
(86) International application number: PCT/US2009/066964
(87) International publication number: WO 2010/077599

(56) References cited:
- US-A- 5 725 552
- US-A- 5 944 738
- US-A1- 2003 212 419
- US-A1- 2005 267 510
- US-A1- 2006 271 099
- US-A1- 2007 265 656
- US-A1- 2007 265 656
- US-B2- 6 599 308

## Description

### BACKGROUND OF THE INVENTION

### I. Field of the Invention:

The present invention generally relates to intravascular devices for treating certain medical conditions and, more particularly, relates to intravascular occlusion devices for selective occlusion of a vessel, channel, lumen or cavity anywhere in the body's circulatory system where it is desired to stop the flow of blood. The devices made in accordance with the invention are particularly well suited for delivery through a small diameter flexible catheter or the like to a remote location treatment site in a patient's vascular system within a patient's body to occlude the site quickly by providing a high metal to volume ratio. The device may have a high ratio of delivery length to deployed length and can reach locations more tortuous than conventional occlusion devices.

### II. Description of the Related Art:

A wide variety of intracardiac prosthetic devices are used in various medical procedures. For example, certain intravascular devices, such as catheters and guide wires, are generally used to deliver fluids or other medical devices to specific locations within the vascular system of a patient, such as a selective coronary artery. Other devices are used in treating specific conditions, such as devices used in removing vascular occlusions or for treating septal defects and the like. For instance, devices have been developed for treating abnormalities, such as an Atrial Septal Defect (ASD), a Ventricular Septal Defect (VSD), a Patent Ductus Arteriosus (PDA), a Patent Foramen Ovale (PFO), Arterial Venous Malformation (AVM), as well as conditions that result from previous medical procedures such as Para-Valvular Leaks (PVL) following surgical valve repair or replacement.

One particular area that has been susceptible to complications is the gonadal vein. The gonadal vein is a collection of veins, wherein the left gonadal vein drains into the left renal vein and the right gonadal vein drains into the inferior vena cava. Incompetent gonadal veins may result in complications for both male and female patients. In particular, varicoceles in male patients and pelvic congestion in female patients can be chronic and painful and may even result in infertility.

Closing the gonadal vein and preventing collateral formation can be used to treat incompetent gonadal veins. Common treatments that may be used include surgical ligation and percutaneous embolization of the gonadal vein. However, ligation may not prevent recurrent complications within the gonadal vein due to collateral flow or failure to ligate branches of the gonadal vein. Embolization overcomes the drawbacks of ligation due to the fact that the entire gonadal vein is occluded. One method of embolizing the gonadal vein is with a coiled device that is positioned within the gonadal vein.

Despite the general ability to occlude the gonadal vein, improving the occlusion within the gonadal vein and reducing the time needed to occlude the gonadal vein is desired so that the device may be accurately and effectively positioned and fixated within the gonadal vein and so that recurrent complications are less likely to occur.

Accordingly, it would be advantageous to provide a reliable medical device that is both easy to deploy through a catheter and that can be accurately placed in a target site such as the gonadal vein. It would also be desirable to provide a low-profile recoverable device for deployment in a target site. Moreover, there is also a need for a medical device that may be effectively fixated within a target site while reducing the incidence of recurrent complications and complications resulting from being fixated therein.

US 2007/0265656 describes multi-layer braided structures for occluding vascular defects. One structure includes two sections having cylindrical or bulbous form which engage a vessel wall and hold the device in place after deployment, with an intermediate clamp clamping an intermediate portion of the multi-braided material between the two sections. Another structure has an outer braid defined volume and an internal smaller diameter braid, pre-shaped into a bead and chain shape, designed to meander into the outer braid defined volume to fill the volume completely.

US 2005/267510 describes an apparatus for embolizing an aneurysm having the shape of porous beads on a coil.

### SUMMARY OF THE INVENTION

The invention provides an occluder device according to claim 1. Preferred embodiments are defined in the dependent claims. More generally the present disclosure describes solutions to the above problems with the prior art. By using a braided tubular fabric made from a shape memory material (e.g., Nitinol) such as that formed by the methods of fabrication, molding and heat treatment and delivery as described by patents 6,123,715 by Amplatz and 5,725,552 by Kotula et al., embodiments of the present disclosure may provide a flexible, low profile vascular occlusion device having a large volume filling capability and high metal content for faster occlusion. As in the referenced patents, braided tubular metal fabric having an expanded preset configuration and an elongated collapsed reduced diameter configuration for delivery through a catheter to a treatment site is shaped to create an occlusion of an abnormal opening in a body organ or vessel. The woven metal fabric has a memory property, whereby the medical device tends to return to said expanded preset configuration when unconstrained. The occlusive device further includes a first shape formed from the braided tubular fabric consisting of a repeating pattern of expanded diameter or volume segments separated by small diameter articulation segments. In one embodiment, the device also includes a second overall device shape comprised of the first shape formed about itself in various volumetric shapes to occlude a vessel or cavity. According to an additional embodiment, an occluder device for occluding a target site is provided. The occluder device includes a tubular structure having proximal and distal ends and a preset, expanded configuration. The tubular structure is configured to be constrained to a reduced configuration for delivery to the target site and to at least partially return to the preset, expanded configuration within the target site when unconstrained, wherein the preset, expanded configuration comprises a plurality of expanded volume members (e.g., 3 or more expanded volume members) and articulating members arranged in a repeating pattern with each articulating member coupling a pair of expanded volume members. In addition, each expanded volume member has a cross-sectional dimension that is larger than a cross-sectional dimension of a respective articulating member, and at least one of the expanded volume members has a cross-sectional dimension at least as large as that of the target site. According to one aspect, the tubular member may be configured to be disposed longitudinally within the target site such that at least one expanded volume member is configured to engage a lumen of the target site. Moreover, each of the expanded volume members may have a cross-sectional dimension at least as large as the target site.

One embodiment of the present disclosure provides a method for delivering an occluder device to a target site. The method includes providing an occluder device comprising a tubular structure having proximal and distal ends and a preset, expanded configuration, wherein the preset, expanded configuration comprises a plurality of expanded volume members and articulating members arranged in a repeating pattern with each articulating member coupling a pair of expanded volume members, and each expanded volume member having a cross-sectional dimension that is larger than a cross-sectional dimension of a respective articulating member. The method also includes constraining the occluder device from the preset, expanded configuration to a reduced configuration and positioning the constrained occluder device in a catheter. Moreover, the method includes delivering the constrained occluder device to the target site and
deploying the constrained occluder device from the catheter such that the occluder device at least partially returns to the preset, expanded configuration within the target site.

According to aspects of the method, the method includes deploying the occluder device such that the occluder device is disposed longitudinally within the target site. The method may also include deploying the occluder device such that at least one expanded volume member engages a lumen of the target site. The method may alternatively include providing an occluder device including a plurality of expanded volume members where in at least one or each of the expanded volume members has a cross-sectional dimension that is at least as large as a cross-sectional dimension of the target site.

An additional embodiment is directed to an occluder device for occluding a gonadal vein. The occluder device includes a tubular structure having proximal and distal ends and a preset, expanded configuration. The tubular structure is configured to be constrained to a reduced configuration for delivery to the gonadal vein and to at least partially return to the preset, expanded configuration within the gonadal vein when unconstrained. The preset, expanded configuration includes a plurality of expanded volume members and articulating members arranged in a repeating pattern with each articulating member coupling a pair of expanded volume members, wherein each expanded volume member has a cross-sectional dimension that is larger than a cross-sectional dimension of a respective articulating member. The tubular member may be configured to be disposed longitudinally within the gonadal vein such that at least one expanded volume member is configured to engage the gonadal vein, although each of the expanded volume members may be configured to do so.

According to another embodiment, a method is provided for delivering the device described above for occluding the gonadal vein. The method includes constraining the occluder device from the preset, expanded configuration to a reduced configuration and positioning the constrained occluder device in a catheter. The method further includes delivering the constrained occluder device to the gonadal vein and deploying the constrained occluder device from the catheter such that the occluder device at least partially returns to the preset, expanded configuration within the gonadal vein. The deploying step may include deploying the occluder device such that the occluder device is disposed longitudinally within the gonadal vein. The method may also include deploying the occluder device such that at least one expanded volume member engages the gonadal vein, although each of the expanded volume members may be configured to do so.

In another embodiment, a shaping wire is contained coaxially within the tubular braid, and provides or assists in the formation of the second overall final device shape.

Embodiments of the present disclosure may be well suited for the selective occlusion of a vessel, lumen, channel, or cavity. Several examples, without limitation, are an aneurysm, a left atrial appendage for patients with left atrial fibrillation, an Arterial Venous Fistula (AVF) or an Arterial Venous Malformation (AVM) or any vessel needed to be occluded to prevent blood flow there through. Other possibilities are treatment of an Atrial Septal Defect (ASD), a Ventricular Septal Defect (VSD), a Patent Foreman Ovale (PFO), or a Patent Ductus Arteriosus (PDA).

When forming these intravascular occlusive devices from a resilient metal fabric, a plurality of resilient strands is provided, with the wires being formed by a braiding machine to create a resilient material which can be heat treated to substantially set a desired shape. This braided fabric is then deformed to generally conform to a molding surface of a molding element and the braided fabric is heat treated in contact with the surface of the molding element at an elevated temperature to form a first molded shape. The first molded shape in space in the preferred embodiment is a repeating expanded diameter or volume segment with an ovaloid, spherical, or disk shape and with a separation between adjacent expanded volumes consisting of the braid formed in a small diameter to function as articulation segments between the expanded volumes. The time and temperature of the heat treatment is selected to substantially set the braided fabric in its deformed state. After the heat treatment, the fabric is removed from contact with the molding element and will substantially retain its shape in an unstressed state. The elongate molded element is further configured to a second heat treated shape by winding the elongate element about itself into any one of a number of three dimensional shapes to be further discussed in the following detailed description. The braided fabric heat treated a second time defines an expanded state of a medical device which can be deployed through a catheter into a channel in a patient's body and placed at a desired target site.

In another embodiment of the disclosure the first heat treatment braid shape is achieved as previously described above, but the final device shape is achieved by use of a shape memory wire sized to be placed through the first shaped heat treatment braid and heat treated as an assembly in a second shape desired of the final device braid central axis. In this embodiment, the wire is inserted into the first shaped heat treated braid prior to the second heat treatment and the distal end of the braid is attached to the distal end of the wire at the braid distal end clamp. The proximal end of the wire free floats within the proximal braid. During use, when the device is pushed out the distal end of a delivery catheter, the shaped wire and braid will assume its memorized final device shape. Alternatively, the shape memory wire may be heat treated separately in the final device or second shape and then inserted into the braid having a first shape. The composite device will take on the final device's second shape based on the shaped wire being stronger in shape retention than the articulation segments of the braid.

Embodiments of the present disclosure provide specific shape improvements over prior art medical devices which may be made in accordance with the present disclosure to address occlusion of vessels having specific anatomical conditions. Such devices of the disclosure are formed of a braided metal fabric and have an expanded configuration and a collapsed configuration. In use, a catheter can be positioned in a channel in a patient's body and advanced to position the distal end of the catheter adjacent a treatment site for treating a physiological condition. A medical device, formed in a predetermined shape, and made in accordance with the process outlined above, can be collapsed by stretching the ends apart and can be inserted into the lumen of the catheter. In use, the device is urged through the catheter and out the distal end, whereupon, due to its memory property, it will tend to substantially return to its expanded state adjacent the treatment site.

In accordance with a first of these embodiments, the occlusive device is shaped into a helix or coil with a length to give it longitudinal stability and adequate anchoring within a vessel or cavity. The device is sized somewhat larger than the vessel or cavity for which it is intended to provide an outward expansion force against the wall of the vessel or cavity to retain the device in place and prevent device embolization. Alternative shapes involve a coil inside a coil, to give a more solid filling of the vessel or alternatively a
device shaped into coils that alternate in diameter from small to larger. Another alternative is a device final shape that is somewhat spherical. There are few, if any, limitations to the shape of device that can be fabricated. Devices may be fabricated to take the shape of an aneurysm of any size and shape. Alternatively, it can be sized to fit a left atrial appendage or other vascular anomaly.

One embodiment provides a means for over the wire delivery while the occlusive device is within the delivery catheter.

The inventive occlusive device will occlude a vessel, channel, lumen, or cavity quickly due the high metal to volume filled ratio and due to the volume occupying expanded diameter portions of the braid that interface with the blood and also restrict blood flow. The device can be stretched for delivery through a small diameter catheter and, due to its flexible nature, can be passed easily through tortuous pathways within the human body.

### BRIEF DESCRIPTION OF THE DRAWINGS

Having thus described the invention in general terms, reference will now be made to the accompanying drawings, which are not necessarily drawn to scale, and wherein:
Figure 1 is a side view of a section of a braided tubular member of a shape memory wire construction prior to its being shaped and heat treated;
Figure 2A is a side view of one example of the tubular member of Figure 1 after shaping in a mold and heat treatment to set memory;
Figure 2B is a side view of another example of the tubular member of Figure 1 after shaping in a mold and heat treatment;
Figure 3A is a side view of a device in a simple coiled shape;
Figure 3B is a side view of a shape memory wire that may be used as an alternative embodiment component to create the shape as illustrated in figure 3A;
Figure 4A is side view of an alternative embodiment of the device having a coiled shape consisting of alternating smaller and larger diameter coils;
Figure 4B is a side view of a shape memory wire that may be used as an alternative embodiment component to create the shape as illustrated in figure 4A;
Figure 5A is a side view of an alternative embodiment of the device having a continuous coil with a small diameter on the inside of the device wrapped by a larger diameter coil;
Figure 5B is a side view of a shape memory wire that may be used as an alternative embodiment component to create the shape as illustrated in figure 5A;
Figure 6A is a side view of the device in a shape approximating a sphere;
Figure 6B is a side view of a shape memory wire that may be used as an alternative embodiment component to create the shape as illustrated in figure 6A;
Figure 7 is a partial cross-sectional view of an alternative embodiment of the inventive device including a shaped memory wire internal to the braid to assist in shaping the device;
Figure 8 is a partial cross-sectional view of another alternative embodiment of the inventive device adapted for over the wire delivery;
Figure 9 is a cross-sectional view of an occluder partially extending from the distal end of a delivery catheter filling a thoracic aortic aneurysm;
Figure 10 is a side view of a medical device in a relaxed configuration according to another embodiment of the present invention;
Figure 11 is a side view of the medical device of Figure 10 positioned within a gonadal vein; and
Figures 12-16 illustrate side views of medical devices in respective relaxed configurations according to another embodiment of the present invention.

### DETAILED DESCRIPTION OF THE PREFERRED EMBODIMENTS

The present invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. Like numbers refer to like elements throughout.

Embodiments of the present invention provide an improved percutaneous catheter directed intravascular occlusion device for use in the vasculature in patients' bodies, such as blood vessels, channels, lumens, a hole through tissue, cavities and the like. In forming
a medical device of the invention, a fabric may be formed of a plurality of wire strands having a predetermined relative orientation between the strands.

The device may include one or more layers of occlusive material, wherein each layer may be comprised of any material that is configured to substantially preclude or occlude the flow of blood so as to facilitate thrombosis. As used herein, "substantially preclude or occlude flow " shall mean, functionally, that blood flow may occur for a short time, but that the body's clotting mechanism or protein or other body deposits on the occlusive material results in occlusion or flow stoppage after this initial time period. For instance, occlusion may be clinically represented by injecting a contrast media into the upstream lumen of the device and if little or no contrast media flows through the device wall after a predetermined period of time, then the position and occlusion of the device is adequate as would be recognized by one of ordinary skill in the art.

As used herein the term "proximal" shall mean closest to the operator (less into the body) and "distal" shall mean furthest from the operator (further into the body). In positioning of the medical device from a downstream access point, distal is more upstream and proximal is more downstream.

According to one embodiment, the occlusive material is a metal fabric including a plurality of strands, such as two sets of essentially parallel generally helical strands, with the strands of one set having a "hand", i.e., a direction of rotation, opposite that of the other set. This defines a generally tubular fabric, known in the fabric industry as a tubular braid. The Amplatz and Kotula patents previously discussed describe medical devices and the methods of fabrication of such devices in great detail so only general discussion is provided.

Although the term "strand" is discussed herein, "strand" is not meant to be limiting, as it is understood the fabric may comprise one or more wires, cords, fibers, yarns, filaments, cables, threads, or the like, such that these terms may be used interchangeably.

The pitch of the wire strands (i.e., the angle defined between the turns of the wire and the axis of the braid) and the pick of the fabric (i.e., the number of wire crossovers per unit length) may be adjusted as desired for a particular application. The wire strands of the metal fabric used in the present method may be formed of a material which is both resilient and which can be heat treated to substantially set a desired shape. Materials which are suitable for this purpose include a cobalt-based low thermal expansion alloy referred to in the field as Elgeloy, nickel-based high temperature high-strength "superalloys" commercially available from Haynes International under the trade name Hastelloy, nickel-based heat treatable alloys sold under the name Incoloy by International Nickel, and a number of different grades of stainless steel. The important factor in choosing a suitable material for the wires is that the wires retain a suitable amount of the deformation induced by the molding surface when subjected to a predetermined heat treatment.

Another class of materials which meet these qualifications is so-called shape memory alloys. One particularly preferred shape memory alloy for use in the present method is a nickel-titanium alloy called Nitinol®. NiTi alloys are also very elastic--they are said to be "superelastic" or "pseudoelastic". This elasticity will help a device of the invention return to a present expanded configuration for deployment when no longer constrained, say, in a lumen of a delivery catheter.

In forming a medical device in keeping with the invention, an appropriately sized piece of the metal fabric is cut from the larger piece of fabric which is formed, for example, by braiding wire strands to form a long tubular braid. When cutting the fabric to the desired dimensions, care should be taken to ensure that the fabric will not unravel.

One can solder, braze, weld or otherwise affix the ends of the desired length together (e.g., with a biocompatible cementitious organic material) before cutting the braid.

Once an appropriately sized piece of the fabric is obtained, the fabric is deformed to generally conform to a surface of a molding element. Deforming the fabric will reorient the relative positions of the strands of the metal fabric from their initial order to a second, reoriented configuration. The shape of the molding element should be selected to deform the fabric into substantially the desired shape.

Once the molding element is assembled with the fabric generally conforming to a molding surface of that element, the fabric can be subjected to a heat treatment while it remains in contact with that molding surface. Suitable heat treatments of Nitinol wire to set a desired shape are well known in the art. It has been found that holding a Nitinol fabric at about 500 degree C to about 550 degree C for a period of about 1 to about 30 minutes, depending on the softness or harness of the device to be made, will tend to set the fabric in its deformed state, i.e., wherein it conforms to the molding surface of the molding element. At lower temperatures the heat treatment time will tend to be greater (e.g. about one hour at about 350 degree C) and at higher temperatures the time will tend to be shorter (e.g. about 30 seconds at about 900 degree C). After the heat treatment, the fabric is removed from contact with the molding element and will substantially retain its shape after being deformed.

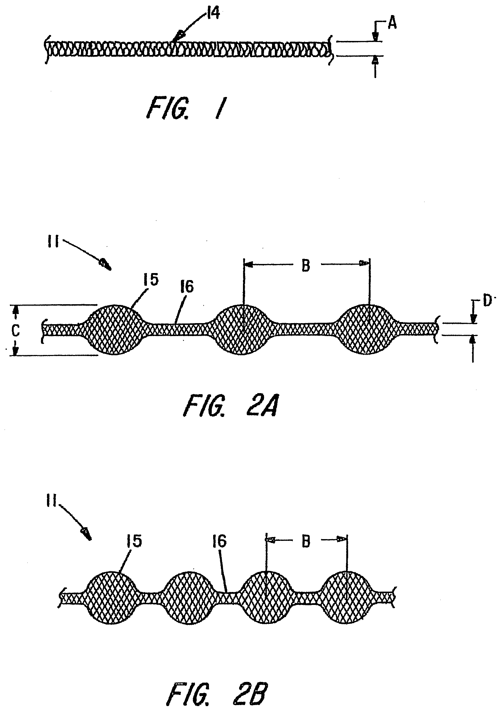

Attention is now directed to Figure 1 which illustrates a braided tube as braided prior to any shape forming a heat treating process. The wires are preferably made of Nitinol alloy and may range from about 0,0254 to 0,1524 mm (0,001 to 0,006 inch) in diameter, preferably about 0,0381 to 0,0762 mm (0,0015 to 0,003 inch) in diameter. The number of wires that make up the braid may be from about 8 to 288, preferably 72-144. The braid may be formed over a mandrel having a diameter approximately the diameter of the expanded volume segment. For example, a device having a 6 mm outer diameter for the expanded volume members may be braided on about a 6 mm diameter mandrel using 144 wires of about 0,0381 mm (0,0015 inch) diameter. The normal gonadal vein may range between about 1-5 mm in inner diameter such that the 6 mm device may be configured to engage the lumen of the gonadal vein. However, the diameter of the gonadal or vericous veins could be as large as about 15 mm and, thus, the expanded volume members may be formed of different sizes for treating different sized veins. The 6 mm device has a profile sufficiently small such that the device may be delivered through a catheter having a lumen diameter of about 1,524 mm (0,060 inch).

Figure 2A illustrates the first heat set shape of the inventive device 11 according to one embodiment of the present invention. The first heat set molded shape consists of the braided tubular member 14 expanded to a larger diameter or volume segment 15 in a repeating pattern with a reduced braid diameter segment 16 between adjacent expanded segments 15. The expanded segments 15 serve as the volume filling aspect of the invention and the reduced diameter portions 16 serve as articulation segments to allow the tubular member to be wound upon itself into various configurations. Figure 2B indicates a pitch B between repeating segments different than that indicated in Figure 2A.

There is no requirement that the original braided tube be made to a specific initial diameter relative to the formed first shape. For example, the initial braided tube could be larger or smaller than the first heat set shape, provided the desired shape is able to be formed and provided the device can be drawn down for insertion into a delivery catheter and the helix angle of the braid in the expanded segment has the desired outward resistance to collapse.

There is also no specific requirement for the pitch between expanded segments or between articulation segments to be uniform. If fact, for certain configurations to be formed in the final device shape it may be highly desirable to alter the spacing of either type of segment at a particular position along the device length as will be discussed later in more detail.

Preferably the expanded segments are either ovaloid, disk, or spherical in shape although they may be any other shape as well, and the expanded shape may have a variable volume or diameter along the device length.

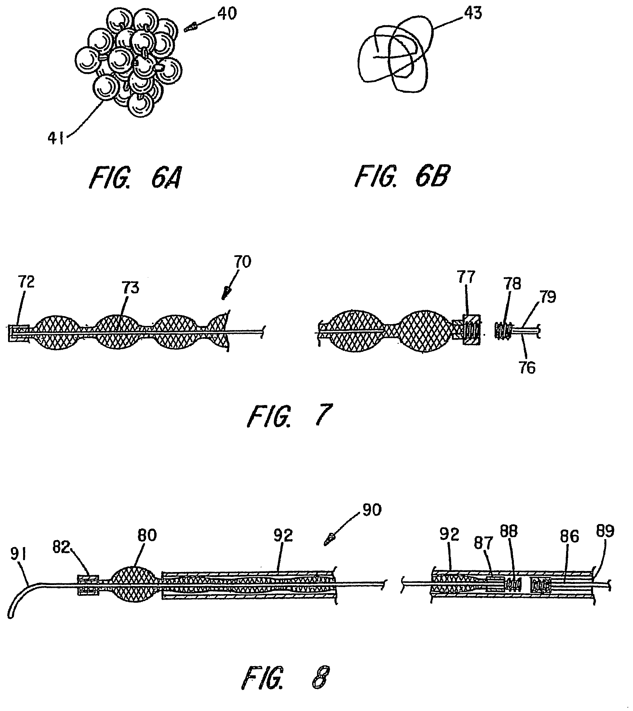

As shown in Figure 7, the ends of this braided metal fabric device 70 may be welded or clamped together with clamps 72 and 77 to avoid fraying. Of course the ends may alternately be held together by other means readily known to those skilled in the art. The clamp 77 tying together the wire strands at the proximal end of device 70 may also serve to connect the device to a delivery system 76. In the embodiment shown, the clamp 77 is generally cylindrical in shape and has a recess for receiving the ends of the metal fabric to substantially prevent the wires comprising the woven fabric from moving relative to one another. The clamp 77 also has a threaded surface within the recess. The threaded recess is adapted to receive and engage the threaded distal end 78 of a delivery device 76. The distal clamp 72 also has a recess for receiving the distal wire ends. The clamps may optionally be fabricated from a radiopaque material such as platinum-iridium alloy or may be stainless steel or other well known materials.

Figure 8 represents an occluder device adapted for over-the-wire delivery. Optionally, but not considered a requirement, the device 90 as shown in FIGURE 8, could be configured with clamp member assemblies 82 and 87 at both wire ends whereby the assembly comprises a hollow inner and outer sleeve. The distal outer clamp member is sized with an inside diameter sufficient to accommodate the braid wire ends surrounding the inner clamp member prior to swaging or alternatively may be bonded between the clamp members or welded in place. The inner clamp member is tubular and is sized with an inside diameter able to freely pass a guidewire 91 therethrough, which typically is about 0,254 to 0,4572 mm (0,010 to 0,018 inch) diameter, preferably 0,254 mm (0,010 inch) wire or cable. The proximal outer clamp member is shown with external threads 88 to reversibly connect to the delivery system 89, which is preferably a nylon block copolymer, such as Pebax®, with a 0,0254 mm (0,001 inch) stainless steel braided wire over the Pebax® inner tube extrusion, followed by another outer layer of Pebax® to cover the braid. Such construction is typical in intravascular guide catheters where flexibility and torque transmission are needed. The Pebax® inside diameter is sufficient to easily pass the guidewire 91.

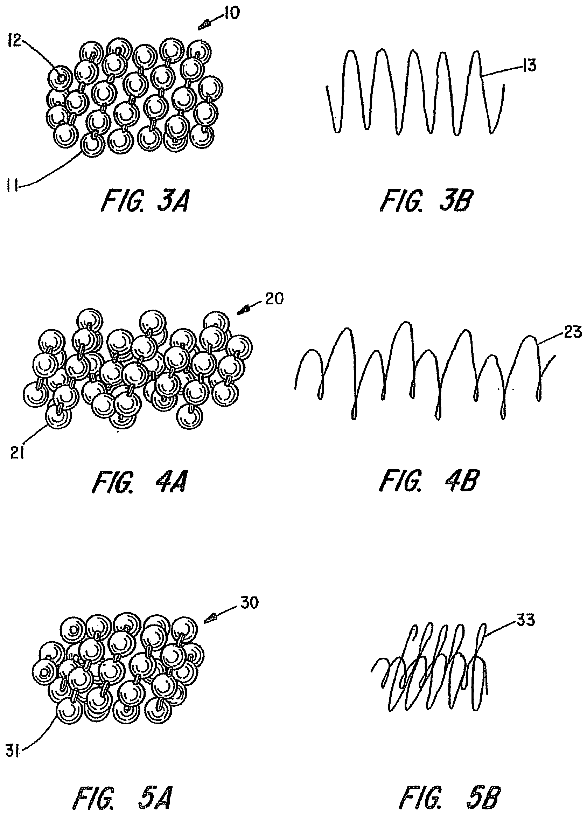

Attention is next directed to Figures 3-6A and 6B. Discussion now is in regard to a heat set second or final device shape. Four examples (no limitation) of specific device final shapes are discussed, as well as three main methods of device fabrication, according to various embodiments of the present invention. In the first general method of forming the second or final device shape, the device of, for example Figure 2A which has a first formed shape that is wrapped about itself into a second or final device shape and held in place during a second heat setting process to retain this shape upon cooling and removing from the mold.

In a second general method of device fabrication, a separate shape memory wire 73 (Figure 7) of preferably between about 0,127 to 0,254 mm (0,005 to 0,010 inch) diameter Nitinol alloy (range of about 0,0762 to 0,508 mm (0,003 to 0,020 inch)) is placed coaxially within the braid having a first formed shape, and the braid and wire assembly are placed in a mold which holds the assembly wire in the shape of the axis of the braided device's desired final shape. In this design, either before or optionally after the assembly heat treatment, the wire 73 and the distal braid wire ends are clamped into the distal clamp 72 by crimp, bond, weld, or other well known means. The proximal end of the wire will match the braid length or be just short of the braid length when the braid is in its free expanded state. The proximal end of the wire free floats within the proximal braid portion to allow the braid to lengthen for placement in a delivery catheter as shown in Figure 7.

In a third general method of device fabrication, the shape memory wire may be heat treated separately in a mold constricting the wire to the final device or second shape. After heat treatment has memorized the desired shape into the wire, the braid with its first heat set established shape is threaded over the wire until the distal end of the wire and the distal end of the braid are aligned. The wire 73 and the distal braid end wires are clamped into the distal clamp 72 by crimp, bond, weld or other well-known means. The proximal end of the wire will match the braid length or be just short of the braid length when the braid is in its free expanded state. The proximal end of the wire free floats within the proximal braid portion to allow the braid to lengthen for placement in a delivery catheter *as* shown in Figure 7. The proximal braid wire ends are clamped into the proximal clamp 77. The composite device will take on the final device's second shape based on the shaped wire being stronger in shape retention than the articulation segments of the braid.

In Figure 3A the occlusion device's second shape consists of a coil or spiral winding of the first shape for a given length and diameter. The length should be minimally about twice the diameter for stability within a vessel. This device shape may be made using any of the first, second or third general fabrication methods. Figure 3B illustrates the longitudinal view of the shape of the separate heat set shaping wire 13 that can be used to fabricate the device in 3A using the second or third general fabrication method. Optionally, the ends of the coil can be shaped such that the last few expanded segments 15 are curved in a smaller radius to occlude the entrance and exit of the coil to further restrict flow. The shapes as shown in Figures 3-5 would be particularly suitable for occluding a tubular vessel and have the ability to adapt to a curve or bend in a vessel. The shape in Figure 6 could be used in a vessel, but may be adaptable to occluding a cavity, such as the left atrial appendage (LAA) or perhaps an aneurysm.

In Figure 4A, the occlusion device 20 second shape consists of a coil or spiral winding having alternating small and large diameters of the first shape for a given length. By alternating the diameters a more complete occlusion can be obtained for filling vessels having inside diameters greater than at least twice the diameter of the expanded volume segments 15. The coiled device length should be minimally about twice the diameter for stability within a vessel. This device shape may be made using any of the first, second or third general fabrication methods previously described. Figure 4B illustrates the longitudinal view showing the shape of the separate heat set shaping wire 23 that can be used to fabricate the device in 4A using the second or third general fabrication method. Alternatively the device could have 3 or more alternating diameters for larger diameter vessels in relation to the expanded segment 15 diameter.

In Figure 5A, the occlusion device 30 has a second shape consisting of a complete internal coil surrounded by an external coil. This provides a dense metal arrangement for occlusion of a vessel or cavity such as an aneurysm or left atrial appendage. The structure can be fabricated using any of the 3 general methods previously discussed and would fill vessels having a diameter at least 4 times larger than the expanded segments 15. Figure 5B illustrated a longitudinal view showing the shape of the separate fabrication heat set shaping wire 33 that can be used to fabricate the device in 5A using the second or third general fabrication method. The device can be configured to first deploy the inside coil and then the outside coil or vice versa. Additionally, it may be desirable to deploy both coils distal to proximal by providing a long articulation segment 16 which extends the length of the first coil proximal end to the distal end of the second coil to allow the second coil to also form distal to proximal.

Figure 6A illustrates a shape of an occlusion device 40 which has a second shape that is somewhat spherical in shape. The shape is fabricated by wrapping the device of the first heat set shape about itself into the desired shape and then heat setting the wrapped shape while being held in place by a mold or other retaining means. As mentioned above, the winding pattern can be selected as desired to form a shape such as an ovaloid for perhaps an aneurysm or the shape of a cavity to be filled, such as a left atrial appendage.

Figure 6B illustrates a separate heat shaped wire that may be used to fabricate the device as shown in Figure 6A using the second or third general fabrication method.

Since a great variety of vessels, aneurysm or cavity sizes may need to be filled, the choice of braid wire diameter, number of wires in the braid and the size of the expanded segments can be chosen relative to the vessel size. It would be preferable that the expanded segment be small in relation to the vessel or cavity maximum width. For example, the expanded diameter segment should be in the range of about 0.4 to 0.1 (preferably about 0.3 to 0.2) of the width of the vessel or cavity to be filled. The final size of the device should be sized to be slightly larger than the vessel or cavity to be filled so as exert an outward force of the vessel or cavity wall to adequately retain the device is place.

Depending on the desired final device shape, the articulation segment length may be altered to obtain the closest fit or nesting of the expanded segments against adjacent expanded segments. As shown in Figure 5A, an outer coil rests against an inner coil. The articulation segment length of the second coil may be different from the inner coil to achieve best packing or nesting of expanded segments. This close nesting provides high metal density and improved flow restriction for quick occlusion (thrombosis) of the vessel.

Those skilled in the art will appreciate that in order to speed up the occlusion of the vessel device, the device may be coated with a suitable thrombogenic agent, filled with a polyester fiber or braided with an increased number of wire strands or fabricated from more than one layer of braided fabric. The prior art devices have preferably used a polyester within the braided device. When placed internal to the braid, his fiber can easily collapse with the device for delivery through a catheter. The interwoven fiber, by attachment to the clot, retains the clot firmly within the device as it forms the occlusion.

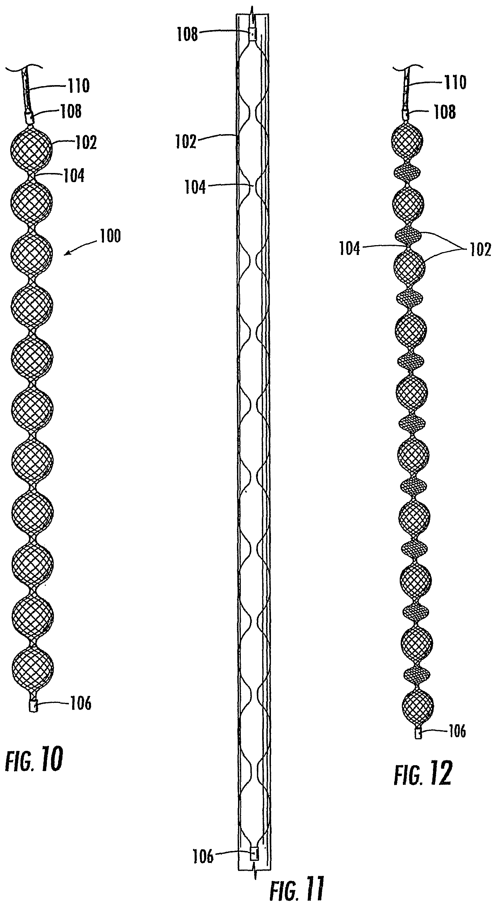

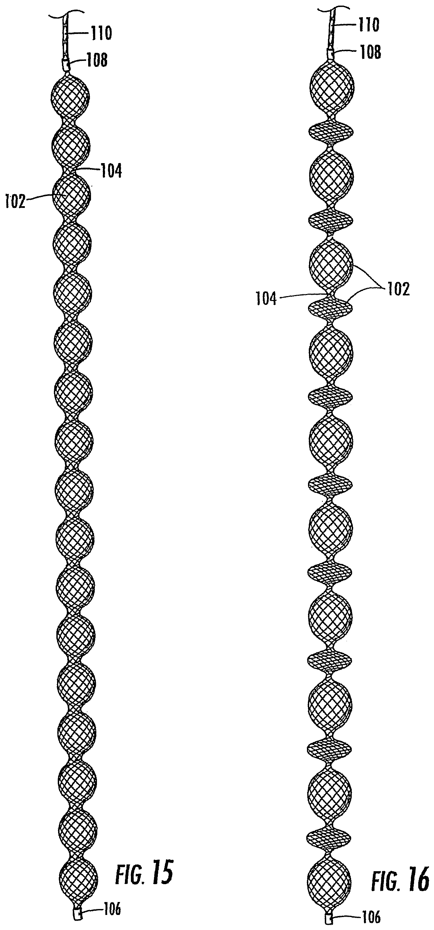

Figures 10 and 11 illustrate an additional embodiment of a medical device 100. The medical device 100 may be used to occlude a variety of target sites such as the gonadal vein. The medical device 100 has a plurality of expanded volume members 102 and articulating members 104 arranged in a repeating pattern. Each articulating member 104 has a smaller cross-sectional dimension than the expanded volume members 102, which may allow the expanded volume members to articulate thereabout and allow the medical device 100 to conform to a variety of contours. Each articulating member 104 is positioned between a pair of expanded volume members 102, and as before, the expanded volume members are typically a sphere, a disk, or an ovaloid shape. However, the expanded volume members 102 could be other shapes if desired (e.g., cylindrical), as long as the expanded volume members have a cross-sectional dimension that is larger than the articulating members 104 and at least as large as that of the target site. For example, FIGS. 12-16 illustrate various configurations of medical devices 100 that include generally spherical, disk, and/or ovaloid shaped expanded volume members 102 according to additional embodiments of the present invention. In addition, there may be any number of expanded volume members and articulating members depending on the target site to be treated. For instance, Figure 10 depicts a medical device 100 having eleven (11) expanded volume members 102 and ten (10) articulating members 104, FIG. 12 shows a medical device having nineteen (19) expanded volume members, and FIG. 15 shows a medical device having sixteen (16) expanded volume members.

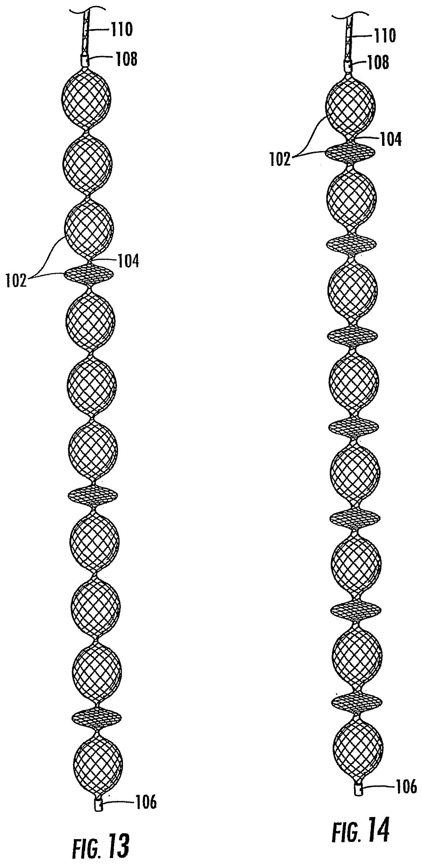

Although each of the expanded volume members 102 is illustrated as being substantially the same size, this need not necessarily be the case. For example, the expanded volume members 102 may be gradually larger or smaller along the device length to engage a target site when the vessel diameter varies in size along its length. In addition, some expanded volume members 102 may be larger than the target site for adequate anchoring while the remaining expanded volume members may be about the same size as the target site. This configuration may be helpful in reducing the device drag through the delivery catheter. FIGS. 12-14 and 16 illustrate various embodiments of medical devices including expanded volume members 102 that have a different shape and/or size from one another. In particular, FIGS. 12 and 14 show alternating generally spherical and disk shaped expanded volume members. Moreover, FIG. 13 shows that there may be different patterns and/or shapes of expanded volume members, wherein in this particular embodiment, the number of spherical volume members is greater than the number of disk shaped members.

As also discussed above, the medical device 100 may be formed of one or more layers of shape memory material that is configured to be elongated or otherwise constrained to a reduced configuration for delivery within a catheter to a target site and assume a preset, expanded configuration upon deployment. According to one embodiment, the medical device 100 includes a male threaded clamp 106 at one end and a female threaded clamp 108 at an opposite end. The threaded clamp 108 may be configured to connect to a delivery device 110 or a threaded connector 106 of a second medical device. Thus, the length of the medical device 100 may be modified by joining a plurality of medical devices together via clamps 106 and 108 depending on the length of the target site to be treated. The male and female threaded connection may be reversed in position as desired or may be replaced by alternative functionally equivalent connectors as are well known in the art so long as the connection between the clamp and the delivery device is reversible.

According to another embodiment, the delivery device may pass through a lumen in the proximal clamp 108 and threadably connect to the distal clamp 106. Another delivery system embodiment comprises an outer tubular shaft which attaches to the proximal end of the occlusion device at clamp 108 and an inner wire or shaft, longer than the outer tubular shaft which passes through the lumen of the outer tubular shaft and threadably engages the distal end of the occlusion device at clamp 106. The medical device may then be elongated for insertion into and passage through the delivery catheter, by advancing the inner wire relative the outer tubular shaft. By pushing on the distal end of the device, the device naturally is decreased in profile and the drag through a catheter is reduced. If desired, a lock between the inner wire and outer tubular shaft may be employed to hold the medical device in its lowest profile, maximum elongated state. In this manner, the inner and outer delivery assembly may be advanced through the delivery catheter together to deliver the device. This system also facilitates retraction of the device back into the catheter or repositioning the device in the target site since the profile of the device can be controlled by relative placement of the delivery inner and outer members. According to one embodiment, this delivery system is configured to deliver a 6 mm diameter by 10cm long device through a 5 French inner diameter catheter.

According to another embodiment, the delivery device need not be threadably, reversably attached to the medical device. A simple pusher delivery device that pushes on either the distal or proximal end of the device may be employed but would be incapable of retrieving the device after deployment out of the delivery catheter.

Figure 11 shows the medical device 100 positioned within a lumen of a target site. At least one expanded volume member 102 or each of the expanded volume members may have a cross-sectional dimension that is at least as large as the cross-sectional dimension of the target site to be treated. For instance, the medical device 100 may be employed to treat a gonadal vein such that each expanded volume member 102 has at least as large a cross-sectional dimension as that of the gonadal vein, as shown in Figure 11. Thus, the expanded volume members 102 are sized and dimensioned to engage the gonadal vein in the preset, expanded configuration and fixate the medical device 100 at the target site. According to one exemplary embodiment, the expanded volume members 102 are about 3 to 20 mm in diameter, and the device may be about 5 to 25 cm in length. Moreover, as also shown in Figure 11 the medical device 100 may be positioned longitudinally within the target site to be treated, such as along the length of the gonadal vein, rather than positioned in a coiled or spherical configuration as discussed above with respect to the embodiments shown in Figures 3-6. Thus, the medical device 100 may not have a shaping wire for forming the medical device into a particular shape.

Therefore, although the device will tend to resiliently return to its initial expanded configuration, i.e., its shape prior to being collapsed for passage through the catheter, it should be understood that it might not always return entirely to that shape. For example, it may be desirable that the expanded volume members 102 have a maximum outer diameter in its expanded configuration at least as large as and preferably larger than, the inner diameter of the opening in which it is to be deployed. For instance, the outer diameter of the expanded volume members 102 may be about 10-30% larger than the inner diameter of the opening. Thus, expanded volume members 102 having a diameter of 6 mm could be used to treat a gonadal vein having a diameter of 4-5 mm, while expanded volume members having a diameter of 15 mm may be used to treat a gonadal vein having a diameter of 12-14 mm. If such a device is deployed in a vessel or abnormal opening having a small lumen, engagement with the lumen will prevent the device from completely returning to its expanded configuration. Nonetheless, the device would be properly deployed because it would engage the inner wall of the lumen to seat and retain the device therein.

When employed for treating the gonadal vein, the medical device 100 provides a simpler and more effective device for occlusion. In addition, the device 100 may also reduce the occlusion time, which may allow the device to be more accurately and effectively positioned and fixated within the gonadal vein. Moreover, by extending longitudinally within the gonadal vein, the medical device 100 may prevent collateral formation or recurrent complications due to the number of occlusive surfaces. Although the device described is useful for occlusion of the gonadal vein it should be understood that the device may be sized and configured for occluding a target site, such as any vein, artery, vessel, passageway, or cavity anywhere in the body.

Figure 8 illustrates a delivery device that can be used to urge the occlusion device 10, 20, 30, 40, or 100 through the lumen of a catheter 92 or long introducer sheath for deployment in a target site of the patient's body. When the device is deployed out the distal end of the catheter, the device will still be retained by the delivery device 79 or 89 in Figures 7 or 8 respectively. Once the proper position of the device in the target site is confirmed, the shaft of the delivery device can be rotated about its axis to unscrew the clamp 77, 87, or 108 from the delivery device 79, 89, or 110 respectively.

By keeping the occlusion device attached to the delivery means, the operator is still able to retract the device for repositioning if it is determined that the device is not properly positioned in a first attempt. This threaded attachment will also allow the operator to control the manner in which the device 10 is deployed out of the distal end of the catheter. When the device exits the catheter, it will tend to resiliently return to a preferred final expanded shape which is set when the fabric is heat treated. When the device springs back into this shape, it may tend to act against the distal end of the catheter, effectively urging itself distally beyond the end of the catheter. This spring action could conceivably result in improper positioning of the device. Since the threaded clamp 77 or 87 (Figure 7 or 8) can enable the operator to maintain a hold on the device during deployment, the spring action of the device can be accommodated and the operator can control the deployment to ensure proper positioning.

Figure 7 illustrates an occluder device fabricated by the second or third general method containing the separate heat set device shaping wire 73 fastened to the distal wire end clamp 72 by crimp, swage, adhesive, solder or other means. The proximal end of the wire 73 floats within the braid and is shorter than the braid when stretched for delivery through the catheter 92 (Figure 8). The delivery device 79 has a wire or cable shaft 76 with an adapter 78 at the distal end with male threads that engage with internal threads on the wire clamp 77.

Figure 8 illustrates a partial cut away of system 90 consisting of occlusion device 80 delivery device 89, a guidewire 91 and the delivery catheter 92. In this embodiment the device 80 has been modified in design for over-the-wire delivery. The proximal and distal end clamps 87 and 82 consist of an inner sleeve and outer sleeve. The inner sleeve has an inside diameter sized for a sliding fit over a guidewire. The outer sleeve is sized to fit over the end wires and over the inner sleeve. The end wires are placed between the two sleeves and are crimped, swaged, welded, or bonded in place to keep the wires from unraveling. The proximal clamp 87 contains threads 88 which mate with a threaded connection on the delivery device 89, which is preferably a nylon block co-polymer extruded tube, such as Pebax, reinforced with a about 0,0254 mm (0,001 inch) braided wire embedded in the Pebax during co-extrusion. Such construction is typical in guide catheters where flexibility and torque transmission are need. Optionally, the delivery device may be a stainless steel coil fabricated from round or flat wire having a lumen sufficient to freely pass the guidewire and covered by a thin polymer tubing, such as a shrink wrap tubing, to contain the coil and provide torque transmission with flexibility and push force. A distal threaded connector is attached, such as by welding or other means to the coiled distal end of the delivery device for reversible connection to the occlusive device 80. The coil shaft of the delivery device may be coated with PTFE to reduce friction between the coil and the guidewire. A small diameter hypotube may also be used as the shaft of a delivery device with the inside diameter sized to accept the guidewire passage there through.

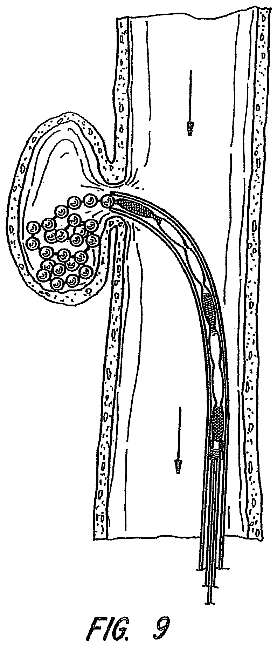

The delivery catheter may be a simple extruded tube preferably made of Pebax. The tubing has a lumen sized for passage of the occlusion device and the delivery device. The delivery catheter may have a shaped tip to allow a predetermined orientation to the occlusion device for delivery to an aneurysm as shown in Figure 9. To improve the torque response of the delivery catheter for tip control, the Pebax tubing may have about 0,0254 mm (0,001 inch) stainless steel wire braided embedded in Pebax polymer, much like a guide catheter. In some cases, a guide catheter itself, or perhaps a diagnostic catheter or long sheath, may serve as the delivery device. According to one embodiment, the medical device may be delivered through a 5 French catheter.

In use, the occlusion device 80 (Figure 8) is connected to the delivery device 89 by threading the devices together. The proximal end of the delivery device 89 is back loaded into the distal end of the delivery catheter 92. The occlusive device 80 is stretched by this motion and drawn down in diameter and into the delivery catheter 92 proximal end. The system 90 may be shipped in this configuration for ease of use reasons. Access (typically via the femoral artery) to the arterial system is achieved by use of the Seldinger Technique as is when known. An arterial sheath is placed in the artery. In the case of an over-the-wire delivery, as shown in Figure 8, a guidewire is first inserted through the sheath and into the artery. The proximal end of the guide wire is back loaded through the occlusion device distal clamp exposed a short amount out the distal end on the delivery catheter. The delivery catheter containing the occlusion device and delivery device is advanced over the guidewire, through the sheath and into the artery. The guidewire is advance and steered through the vasculature to the target location. The delivery catheter is advanced until the distal tip is positioned at the target site. The guide wire may be removed before advancement of the device 80 out the delivery catheter or may be withdrawn after the occlusion device is partially into the vessel or cavity to be filled. The device 80 is then advanced by pushing on the delivery device 89 until the entire device 80 is positioned as desired. If the device is positioned correctly, the delivery device is unscrewed by rotation from the occlusion device and the delivery catheter and delivery device are withdrawn. If the device is not initially positioned correctly, the device 80 may be drawn back into the delivery catheter 92 and a further attempt made to reposition the device as often as needed so long as the threaded connection is retained. Once the threaded connection is separated, the device cannot be recaptured into the catheter 92.

In the case of a non-over-the wire device as shown in Figures 7 and 9, the delivery catheter may be advanced over a guidewire, as long as the occlusive device and attached delivery device are not pre-loaded into the catheter. Once the delivery catheter is positioned as desired the guide wire may be withdrawn from the body and the occlusive device and delivery device must be forward loaded into the proximal end of the delivery catheter. This may be accomplished by placement of a tear-away introducer sleeve over the distal end of the delivery device before connection to the occlusive device. The introducer sleeve has an outside diameter slightly smaller than the I.D. of the delivery catheter. The threaded connection is made between the occlusion device and the delivery device and the occlusive device is drawn into the tear-away sleeve. Once inside the sleeve, the occlusive device can be piloted into the usual Luer connector leading to the lumen on the proximal end of the delivery catheter 92. Advancement of the delivery device delivers the occlusive device through the delivery catheter. The introducer sleeve may now be torn away from the delivery device and discarded. With the delivery catheter in position, the delivery device may be advanced to deliver the occlusive device to the target site for vessel or cavity being occluded.

As discussed above, one embodiment of the present invention is directed to a device for occluding the gonadal vein. Since the gonadal vein origin is either from the left renal vein or the inferior vena cava, access to deliver the device may be made by first placing an access sheath (e.g., Seldinger Technique) and a guide catheter having an appropriate shaped distal tip into the right or left femoral vein. The distal shaped tip provides access to the ostium or the right or left gonadal vein. The delivery catheter and guide wire may be used to advance the delivery catheter through the guide catheter and through the gonadal vein until the target location is reached. The device may then be advanced through the delivery catheter (guidewire removed) to the target site using the delivery system attached to the device. Once the device is at the proper position and deployed from the delivery catheter, the delivery device may be disconnected and all access and delivery catheters removed from the body. If the device is designed for over the wire delivery, it may be preloaded into the delivery catheter over the guidewire and advanced with the delivery catheter to the target site.

Angiography is typically used in catheter based procedures to image the devices and catheters during delivery. The end clamps may be radiopaque or radiopaque markers may be added to the occlusive device if desired. Generally the amount of metal in these occlusive devices can be seen on fluoroscopy. Radiopaque dye may be used to determine when blood flow through the vessel or cavity has been stopped by thrombosis.

Although the device will tend to resiliently return to its initial expanded configuration (i.e., its shape prior to being collapsed for passage through the catheter), it should be understood that it may not always return entirely to that shape. For example, the device is intended to have a maximum outer diameter in its expanded configuration at least as large as and preferably larger than, the inner diameter of the lumen in which it is to be deployed. If such a device is deployed in a vessel having a small lumen, the lumen will prevent the device from completely returning to its expanded configuration. Nonetheless, the device would be properly deployed because it would engage the inner wall of the lumen to seat the device therein, as detailed above.

If the device is to be used to permanently occlude a channel or cavity in the patient's body, such as the devices 10, 20, 30, 40, 70, 80, and 100 described above may be, one can simply disconnect the delivery system by reversing the reversible connection to the device and retract the catheter and delivery device from the patient's body. This will leave the medical device deployed in the patient's vascular system so that it may occlude the blood vessel or other target site in the patient's body.

Many modifications and other embodiments of the invention set forth herein will come to mind to one skilled in the art to which this invention pertains having the benefit of the teachings presented in the foregoing descriptions and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. An occluder device (100) for occluding a target site, the occluder device comprising: a tubular structure having proximal and distal ends and a preset, expanded configuration, the tubular structure having a ratio of length to maximum outer diameter of at least 4 in the preset, expanded configuration, the tubular structure configured to be constrained to a reduced configuration for delivery to the target site and to at least partially return to the preset, expanded configuration within the target site when unconstrained, wherein the preset, expanded configuration comprises a plurality of expanded volume members (102) and articulating members (104) arranged in a repeating pattern, wherein the plurality of expanded members comprise at least one of a spherical member or an ovaloid member, with each articulating member coupling a pair of expanded volume members, and each expanded volume member having a cross-sectional dimension that is larger than a cross-sectional dimension of a respective articulating member, and wherein at least one expanded volume member has a cross-sectional dimension at least as large as that of the target site,
wherein the tubular member is sized and configured to be disposed longitudinally within the gonadal vein such that at least one, or each, expanded volume member (102) is configured to engage the gonadal vein.

2. The occluder device (100) of Claim 1, wherein the expanded volume members (102) further comprise at least one disk.

3. The occluder device (100) of Claim 1, wherein the tubular structure comprises at least one layer of braided fabric.

4. The occluder device (100) of Claim 3, wherein said at least one layer of braided fabric comprises a shape memory alloy.

5. The occluder device (100) of Claim 1, further comprising a pair of end clamps (106, 108), wherein each of the proximal and distal ends is secured with a respective end clamp.

6. The occluder device (100) of Claim 5, wherein at least one of the end clamps comprises a threaded connection configured to be coupled to a delivery device (110) or a second occluder device (100).

7. The occluder device (100) of Claim 1, wherein each of the plurality of expanded volume members (102) has a cross-sectional dimension at least as large as that of the target site.

8. The occluder device (100) of Claim 1, wherein each expanded volume member (102) has an outer diameter of about 3 to 20 mm in the preset, expanded configuration.

9. The occluder device (100) of Claim 1, wherein the tubular structure has a length between its proximal and distal ends of at least about 5 cm in the preset, expanded configuration.

10. The occluder device (100) of Claim 1, wherein the tubular structure has a ratio of length to maximum outer diameter of about 83 in the preset, expanded configuration.

11. The occluder device (100) of Claim 1, wherein each expanded volume member (102) has approximately the same maximum outer diameter in the preset, expanded configuration.

12. The occluder device (100) of Claim 1, wherein at least one expanded volume member (102) has a different shape than another expanded volume member in the preset, expanded configuration.

13. The occluder device (100) of Claim 1, wherein the expanded volume members (102) comprise at least one of gradually larger, or gradually smaller, in size members along the device length.

14. The occluder device (100) of Claim 1, deliverable through a catheter (92) having a 5 French lumen.

15. The occluder device (100) according to any preceding claim, further comprising at least a second layer of fabric comprising a plurality of braided strands.

16. The occluder device (100) of Claim 6, wherein at least one of the pair of end clamps (106, 108) allows passage of a wire or shaft therethrough.

17. The occluder device (100) of claim 1, wherein said tubular structure comprises a chain of expanded volume members alternating in shape along the chain between two or more of spherical, disk-shaped and ovaloid members.

## Patentansprüche

1. Verschlussvorrichtung (100) zum Verschließen einer Zielstelle, wobei die Verschlussvorrichtung Folgendes umfasst:
eine röhrenförmige Struktur mit einem proximalen und einem distalen Ende und einer voreingestellten, erweiterten Anordnung, wobei die röhrenförmige Struktur ein Verhältnis von Länge zum maximalen Außendurchmesser von mindestens 4 in der voreingestellten, erweiterten Anordnung aufweist, wobei die röhrenförmige Struktur dazu konfiguriert ist, um sich auf eine reduzierte Anordnung zur Abgabe an die Zielstelle zu beschränken, und um mindestens teilweise zu der voreingestellten, erweiterten Anordnung innerhalb der Zielstelle zurückzukehren, wenn sie nicht eingeschränkt ist, wobei die voreingestellte, erweiterte Anordnung mehrere erweiterte Volumenelemente (102) und Gelenkelemente (104) umfasst, die in einem sich wiederholenden Muster angeordnet sind, wobei die mehreren erweiterten Elemente ein kugelförmiges Element und/oder ein ovalförmiges Element umfassen, wobei jedes Gelenkelement ein Paar von erweiterten Volumenelementen koppelt und jedes erweiterte Volumenelement eine Querschnittsabmessung aufweist, die größer ist als eine Querschnittsabmessung eines jeweiligen Gelenkelements, und wobei mindestens ein erweitertes Volumenelement eine Querschnittabmessung aufweist, die mindestens so groß ist wie diejenige der Zielstelle, wobei das röhrenförmige Element bemessen und konfiguriert ist, um in Längsrichtung innerhalb der Gonadenvene derart angeordnet zu sein, dass mindestens ein oder jedes erweiterte Volumenelement (102) konfiguriert ist, um mit der Gonadenvene in Eingriff zu kommen.

2. Verschlussvorrichtung (100) nach Anspruch 1, wobei die erweiterten Volumenelemente (102) ferner mindestens eine Scheibe umfassen.

3. Verschlussvorrichtung (100) nach Anspruch 1, wobei die röhrenförmige Struktur mindestens eine Schicht aus geflochtenem Gewebe umfasst.

4. Verschlussvorrichtung (100) nach Anspruch 3, wobei die mindestens eine Schicht aus geflochtenem Gewebe eine Formgedächtnislegierung umfasst.

5. Verschlussvorrichtung (100) nach Anspruch 1, ferner umfassend ein Paar Endklammern (106, 108), wobei jedes der proximalen und distalen Enden mit einer jeweiligen Endklammer gesichert ist.

6. Verschlussvorrichtung (100) nach Anspruch 5, wobei mindestens eine der Endklammern eine Gewindeverbindung umfasst, welche konfiguriert ist, um mit einer Abgabevorrichtung (110) oder einer zweiten Verschlussvorrichtung (100) gekoppelt zu werden.

7. Verschlussvorrichtung (100) nach Anspruch 1, wobei jedes der mehreren erweiterten Volumenelemente (102) eine Querschnittsabmessung aufweist, die mindestens so groß ist, wie die der Zielstelle.

8. Verschlussvorrichtung (100) nach Anspruch 1, wobei jedes erweiterte Volumenelement (102) in der voreingestellten, erweiterten Anordnung einen Außendurchmesser von etwa 3 bis 20 mm aufweist.

9. Verschlussvorrichtung (100) nach Anspruch 1, wobei die röhrenförmige Struktur eine Länge zwischen ihrem proximalen und distalen Ende von mindestens etwa 5 cm in der voreingestellten, erweiterten Anordnung aufweist.

10. Verschlussvorrichtung (100) nach Anspruch 1, wobei die rohrförmige Struktur ein Verhältnis von Länge zum maximalen Außendurchmesser von etwa 83 in der voreingestellten, erweiterten Anordnung aufweist.

11. Verschlussvorrichtung (100) nach Anspruch 1, wobei jedes erweiterte Volumenelement (102) in der voreingestellten, erweiterten Anordnung ungefähr den gleichen maximalen Außendurchmesser aufweist.

12. Verschlussvorrichtung (100) nach Anspruch 1, wobei mindestens ein erweitertes Volumenelement (102) eine andere Form als ein anderes erweitertes Volumenelement in der voreingestellten, erweiterten Anordnung aufweist.

13. Verschlussvorrichtung (100) nach Anspruch 1, wobei die erweiterten Volumenelemente (102) mindestens eines von Elementen mit allmählich größerer oder allmählich kleinerer Größe entlang der Vorrichtungslänge umfassen.

14. Verschlussvorrichtung (100) nach Anspruch 1, welche durch einen Katheter (92) mit einem Lumen von 5 French zugeführt werden kann.

15. Verschlussvorrichtung (100) nach einem der vorhergehenden Ansprüche, ferner umfassend wenigstens eine zweite Schicht aus Gewebe mit mehreren geflochtenen Strängen.

16. Verschlussvorrichtung (100) nach Anspruch 6, wobei mindestens eine der beiden Endklammern (106, 108) den Durchgang eines Drahtes oder eines Schafts ermöglicht.

17. Verschlussvorrichtung (100) nach Anspruch 1, wobei die röhrenförmige Struktur eine Kette von erweiterten Volumenelementen umfasst, deren Form sich entlang der Kette zwischen zwei oder mehreren kugelförmigen, scheibenförmigen und ovalförmigen Elementen abwechselt.

## Revendications

1. Dispositif d'occlusion (100) destiné à occlure un site cible, le dispositif d'occlusion comprenant :
une structure tubulaire comportant des extrémités proximale et distale et une configuration étendue prédéfinie, la structure tubulaire présentant un rapport de la longueur au diamètre extérieur maximal d'au moins 4 dans la configuration étendue prédéfinie, la structure tubulaire étant conçue pour être contrainte à une configuration réduite pour livraison au site cible et retour au moins partiel à la configuration étendue prédéfinie à l'intérieur du site cible sans contrainte, dans lequel la configuration étendue prédéfinie comprend une pluralité d'éléments de volume étendus (102) et d'éléments d'articulation (104) disposés dans un motif récurrent, dans lequel la pluralité d'éléments étendus comprennent au moins un élément sphérique ou ovaloïde, chaque élément d'articulation accouplant une paire d'éléments de volume étendus et chaque élément de volume ayant une dimension de section transversale supérieure à celle d'un élément d'articulation respectif, et dans lequel au moins un élément de volume étendu a une dimension de section transversale au moins aussi grande que celle du site cible, dans lequel l'élément tubulaire est dimensionné et configuré pour être disposé longitudinalement à l'intérieur de la veine gonadique, de sorte qu'au moins un élément, ou chaque élément de volume étendu (102) soit configuré pour venir en prise avec la veine gonadique.

2. Dispositif d'occlusion (100) selon la revendication 1, dans lequel les éléments de volume étendus (102) comprennent en outre au moins un disque.

3. Dispositif d'occlusion (100) selon la revendication 1, dans lequel la structure tubulaire comprend au moins une couche de tissu tressé.

4. Dispositif d'occlusion (100) selon la revendication 3, dans lequel ladite au moins une couche de tissu tressé comprend un alliage à mémoire de forme.

5. Dispositif d'occlusion (100) selon la revendication 1, comprenant en outre une paire de pinces d'extrémité (106, 108), dans lequel chacune des extrémités proximale et distale est fixée à une pince d'extrémité respective.

6. Dispositif d'occlusion (100) selon la revendication 5, dans lequel au moins l'une des pinces d'extrémité comprend une liaison filetée configurée pour être accouplée à un dispositif de distribution (110) ou à un second dispositif d'occlusion (100).

7. Dispositif d'occlusion (100) selon la revendication 1, dans lequel chacun de la pluralité d'éléments de volume étendus (102) a une dimension de section transversale au moins aussi grande que celle du site cible.

8. Dispositif d'occlusion (100) selon la revendication 1, dans lequel chaque élément de volume étendu (102) présente un diamètre extérieur d'environ 3 à 20 mm dans la configuration étendue prédéfinie.

9. Dispositif d'occlusion (100) selon la revendication 1, dans lequel la structure tubulaire présente une longueur entre ses extrémités proximale et distale d'au moins environ 5 cm dans la configuration étendue prédéfinie.

10. Dispositif d'occlusion (100) selon la revendication 1, dans lequel la structure tubulaire présente un rapport de la longueur au diamètre extérieur maximal d'environ 83 dans la configuration étendue prédéfinie.

11. Dispositif d'occlusion (100) selon la revendication 1, dans lequel chaque élément de volume étendu (102) présente approximativement le même diamètre extérieur maximal dans la configuration étendue prédéfinie.

12. Dispositif d'occlusion (100) selon la revendication 1, dans lequel au moins un élément de volume étendu (102) présente une forme différente de celle d'un autre élément de volume étendu dans la configuration étendue prédéfinie.

13. Dispositif d'occlusion (100) selon la revendication 1, dans lequel les éléments de volume étendus (102) comprennent au moins un élément de plus en plus grand ou de plus en plus petit sur la longueur du dispositif.

14. Dispositif d'occlusion (100) selon la revendication 1, pouvant être délivré à travers un cathéter (92) comportant une lumière française de 5.

15. Dispositif d'occlusion (100) selon l'une quelconque des revendications précédentes, comprenant en outre au moins une seconde couche de tissu comprenant une pluralité de brins tressés.

16. Dispositif d'occlusion (100) selon la revendication 6, dans lequel au moins l'une des paires de pinces d'extrémité (106, 108) permet le passage d'un fil ou d'une tige à travers celui-ci.

17. Dispositif d'occlusion (100) selon la revendication 1, dans lequel ladite structure tubulaire comprend une chaîne d'éléments de volume étendus alternant de forme le long de la chaîne entre au moins deux éléments sphériques, en forme de disque et ovaloïdes.
